# EUROPÄISCHE PATENTSCHRIFT

(11) **EP 2 112 508 B1**
(45) Veröffentlichungstag und Bekanntmachung des Hinweises auf die Patenterteilung: **13.10.2010**
(21) Anmeldenummer: 08007779.5
(22) Anmeldetag: 22.04.2008
(51) Int. Cl.: G01N 33/49

(54) **Aggregometer**
Aggregometer
Agrégomètre

(43) Veröffentlichungstag der Anmeldung: 28.10.2009
(73) Patentinhaber: Behnk, Holger, D-22417 Hamburg (DE)
(72) Erfinder: Behnk, Holger, D-22417 Hamburg (DE)
(74) Vertreter: Glawe, Delfs, Moll

(56) Entgegenhaltungen:
- WO-A-2005/059532
- DE-T2- 69 821 364
- US-A- 3 486 859
- US-A- 5 491 408
- US-B1- 7 021 122

## Beschreibung

Die Erfindung betrifft ein Aggregometer zum Messen und Aufzeichnen von Thrombozytenaggregation mit mindestens einer Messküvette, mit einer Einrichtung zum Bewegen der zu untersuchenden Flüssigkeit und mit einer Mess- und Auswertungselektronik, wobei die Messküvette zwei nebeneinander angeordnete, oben offene Teilräume enthält, die nur im unteren Bereich miteinander verbunden sind, und die Einrichtung zum Bewegen der Flüssigkeit eine dichtend auf einen der Teilräume aufsetzbare periodisch und abwechselnd einen Unterdruck/Überdruck erzeugende Pumpeinrichtung ist.

Ein bekanntes Aggregometer (DE 698 21 364 T2) dient dazu, Thrombozytenaggregation zu messen und aufzuzeichnen. Blut weist in seinem proteinreichen Fluid, dem Plasma, drei Hauptgruppen von Zellen auf, nämliche rote Blutkörperchen, weiße Blutkörperchen und Thrombozyten. Thrombozyten können einer Aggregations-Adhäsions-Reaktion ausgesetzt werden, wenn sie mit bestimmten Materialen und Chemikalien in Kontakt gelangen. Sie ändern dann ihre Form und werden ungefähr kugelförmig. Dabei entwickeln sie lange Fortsätze, wodurch sie klebrig werden. Die Thrombozyten kleben dann aneinander und an beschädigtem Gewebe.

Diese Aggregation spielt z. B. bei Operationen eine große Rolle. Man kann die Aggregation durch gewisse Medikamente verringern, ist aber darauf angewiesen, zu wissen, ob bei einer gewissen Medikamentdosis tatsächlich die Aggregation vermindert wird, da unterschiedliche Menschen unterschiedlich auf die Medikamente reagieren.

Bei einem bekannten Verfahren wird die Aggregationsreaktion in Proben von thrombozytenreichen Plasma durch Messung der Lichtdurchlässigkeit durch die Probe analysiert. Hierzu ist es aber notwendig, dass Blut aufwändig mittels Zentrifugation zu trennen, wodurch die Eigenschaften der Thrombozyten verfälscht werden können.

Dieser Nachteil wird im genannten Stand der Technik dadurch vermieden, dass Blut, das zwei Elektroden bedeckt, gerührt wird. Setzen sich die Thrombozyten auf den Elektroden ab, so wird der elektrische Widerstand, der gemessen wird, anwachsen, so dass man auf diese Weise die Aggregation feststellen kann. Im Gegensatz zu anderen Verfahren kann dabei auch die Messung mit Blut erfolgen; eine Zentrifugierung ist nicht erforderlich.

Nachteile dieses bekannten Aggregometers bestehen einmal darin, dass die Elektroden biegsam sind, so dass die elektrischen Eigenschaften für die Messung nicht konstant sind. Der Rührstab, der zum Bewegen des Bluts verwendet wird, wird das Blut lokal in seiner Nähe sehr stark bewegen, während es in den Bereichen, die weiter vom Rührstab entfernt sind, weniger bewegt wird. Beides entspricht nicht den physiologischen Vorgängen im menschlichen oder tierischen Körper.

Bei weiteren bekannten Aggregometern werden ebenfalls Rühreinrichtungen (WO 2005/059532) oder Vibrationseinrichtungen (US 5,491,408) im Zusammenhang mit stab- oder drahtförmigen Elektroden verwendet.

Bei einem Aggregometer der eingangs genannten Art werden periodische Druckänderungen zum Bewegen der zu untersuchenden Flüssigkeit verwendet; die Messung erfolgt über die Überwachung der Druckänderungen (US 3,486,859).

Die Aufgabe der Erfindung besteht daher in der Schaffung eines Aggregometers mit Verwendung von Elektroden für die Messung, bei dem die Messung bei einer gleichmäßigeren Blutbewegung durchgeführt werden kann, wobei die elektrischen Eigenschaften der Elektroden nicht variieren.

Die erfindungsgemäße Lösung besteht darin, dass die Messküvette zwei Elektroden aufweist, die als in den Boden der Messküvette eingesetzte Metallkugeln ausgebildet sind und dass die Mess- und Auswertungselektronik über Kontaktelemente mit den Elektroden verbunden ist.

Die Messküvette enthält zwei nebeneinander angeordnete oben offene Teilräume. Diese sind im unteren Bereich miteinander verbunden. Die Elektroden sind dabei als in den Boden der Messküvette eingesetzte Metallkugeln ausgebildet, die konstanten Abstand und unveränderbare Oberflächen haben, so dass die elektrischen Eigenschaften für die Messungen immer konstant und dieseleben sind. Auf einen der Teilräume wird dann im Wesentlichen dichtend eine Pumpeinrichtung aufgesetzt, die in diesem Teilraum periodisch und abwechselnd einen leichten Unterdruck und Überdruck erzeugt. Dadurch wird die Flüssigkeit (z. B. das Blut) von dem einen Teilraum in den anderen Teilraum hin- und zurückbewegt und überstreicht dabei jeweils die Kugeln. Diese Bewegung, die einen gleichmäßigen Blutstrom bedeutet, entspricht dabei im Wesentlichen auch dem Fließverhalten des Blutes im menschlichen und tierischen Körper.

Zweckmäßigerweise sind die Metallkugeln in den Boden der Küvette eingegossen oder eingepresst. Sie bestehen zweckmäßigerweise aus einem nicht rostenden Material, insbesondere Edelstahl. Andererseits können die Metallkugeln aber auch mit einem nichtrostenden Material, insbesondere einem Edelmetall wie z.B. Gold beschichtet sein.

Bei einer anderen vorteilhaften Ausführungsform sind die Metallkugeln mit einem Reagenz beschichtet.

Wenn die Pumpgeschwindigkeit veränderbar ist, kann man unterschiedliche Strömungsgeschwindigkeiten erreichen.

Zweckmäßigerweise weist die Pumpe einen Zylinder und einen Kolben auf, der durch einen Exzenterantrieb hin- und her bewegbar ist. Durch Verändern der Geschwindigkeit des Exzenterantriebs kann dann die Pumpfrequenz oder Pumpgeschwindigkeit verändert werden.

Zweckmäßigerweise weist der Teilraum, auf den die Pumpeinrichtung aufsetzbar ist, im Wesentlichen parallele Wände auf. Der andere Teilraum erweitert sich aber nach oben hin zweckmäßigerweise trichterförmig, um so das Einführen der Blutprobe zu erleichtern.

Zweckmäßigerweise sind die Kontaktelemente, mit denen die Metallkugeln mit der Messelektronik verbunden werden, federnd ausgebildet. Dies erleichtert das Auswechseln von verschiedenen Küvetten bzw. das Einsetzen in eine Messstation.

Dies ist besonders vorteilhaft, wenn mehrere Messküvetten nebeneinander angeordnet sind. Dabei können Einrichtungen vorgesehen sein, so dass die einzelnen Küvetten gleichzeitig mit der Mess- und Auswerteelektronik verbunden werden können, indem die Metallkugeln mithilfe ihrer Kontaktelemente mit der Elektronik verbunden werden und wobei eine oder mehrere Pumpeinrichtungen auf die Messküvetten aufgesetzt und betrieben werden.

Die Messküvetten können als Einwegteile hergestellt werden. Besonders zweckmäßig ist es, wenn sie Spritzgussteile aus blutverträglichen Kunststoffmaterialien sind, die aus Polystyrol, Polymethylmethacrylat, Polyethylen und ähnlichen Kunststoffen ausgewählt sind.

Die Erfindung wird im Folgenden anhand von vorteilhaften Ausführungsformen unter Bezugnahme auf die beigefügten Zeichnungen bspw. beschrieben. Es zeigen:
- Fig. 1: schematisch den Aufbau des erfindungsgemäßen Ag- gregometers in einer ersten Betriebsphase;
- Fig. 2: den Aufbau des Aggregometers in einer zweiten Be- triebsphase;
- Fig. 3: eine andere Ausführungsform der Messküvetten in perspektivischer Ansicht; und
- Fig. 4: die Messküvetten von Fig. 3 in Draufsicht von o- ben.

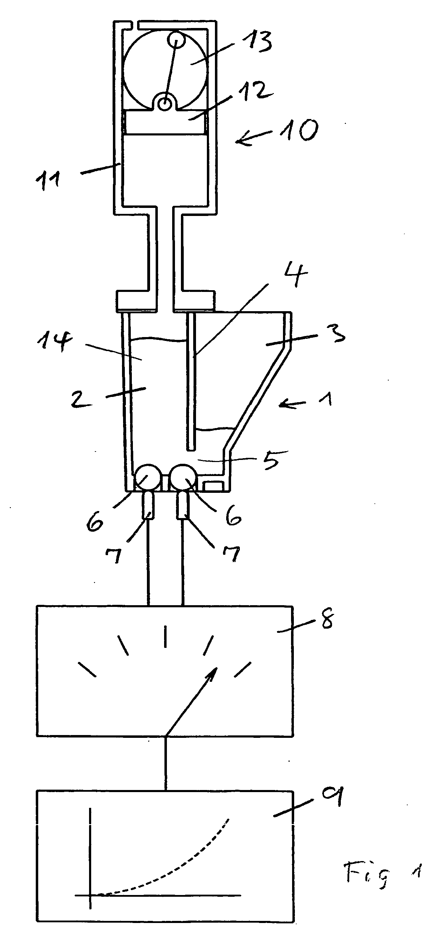

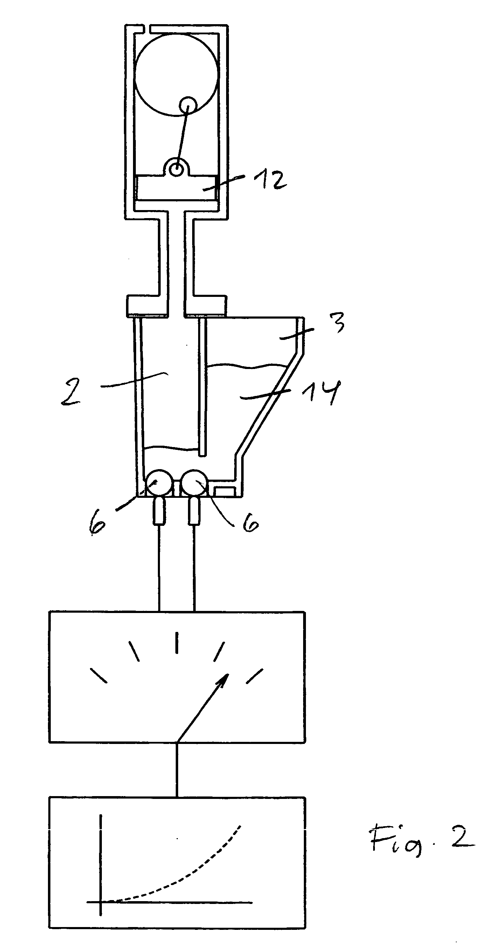

Wie dies in Fig. 1 dargestellt ist, weist die Messküvette 1 zwei Teilräume 2 und 3 auf. Der Teilraum 2 hat dabei im Wesentlichen parallele Wände, während sich der Teilraum 3 trichterförmig nach oben erweitert. Die beiden Teilräume 2 und 3 sind durch eine Zwischenwand 4 getrennt, die nur im unteren Bereich einen Durchlass 5 freilässt. Hier sind zwei Metallkugeln 6 vorgesehen, insbesondere in den Boden eingegossen oder eingepresst, die über federnde Kontaktelemente 7 mit der Messelektronik 8 verbunden sind, deren Messergebnisse z. B. bei 9 grafisch dargestellt werden können. Auf den ersten Teilraum 2 der Messküvette 1 ist eine Pumpeinrichtung 10 im Wesentlichen dichtend aufgesetzt, die einen Zylinder 11 mit einem Kolben 12 aufweist, der über einen Exzenterantrieb 13 auf- und ab bewegt werden kann. In der in Fig. 1 dargestellten Betriebsstellung befindet sich der Kolben 12 oben und saugt dadurch das Blut 14 im ersten Teilraum 2 an. Bei der Betriebsstellung der Fig. 2 befindet sich der Kolben 12 unten und drückt dadurch das Blut 14 in den zweiten Teilraum 3. Bei dieser Hin- und Herbewegung überstreicht das Blut 14 die Metallkugeln 6. Findet eine Aggregation von Thrombozyten statt, erhöht sich der elektrische Widerstand, der zwischen diesen beiden Elektroden 6 gemessen wird. Es ist daher möglich, dass Aggregationsverhalten des Blutes zu messen, insbesondere auch in Abhängigkeit vom verabreichten Medikament. Man kann so z. B. feststellen, ob und wie ein spezielles Medikament (z. B. Acetylsalizylsäure) wirkt.

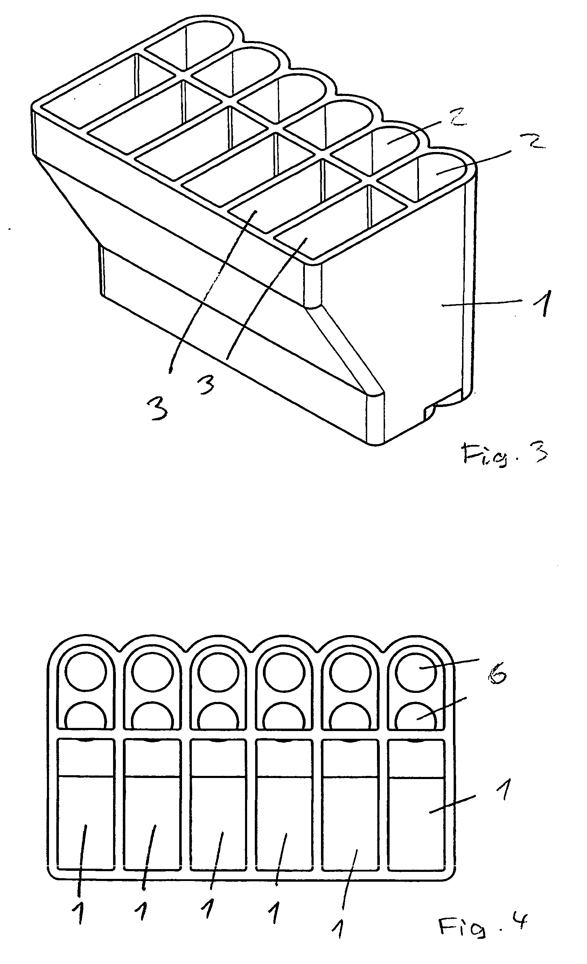

Die Messung kann dabei sowohl im Labor als auch im Operationssaal durchgeführt werden. Sie kann auch automatisiert werden. Dies ist insbesondere der Fall, wenn eine Vorrichtung gemäß Fig. 3 verwendet wird, wo mehrere Küvetten 1 nebeneinander angeordnet sind. Die einzelnen Küvetten 1 können dann gleichzeitig mithilfe der federnden Kontakte 7 mit der Messelektronik 8 verbunden werden. Nachdem dann die Pumpeinrichtung 10 oder mehrere Pumpeinrichtungen auf die Küvetten aufgesetzt sind, kann die Messung gleichzeitig für alle Küvetten durchgeführt werden.

## Patentansprüche

1. Aggregometer zum Messen und Aufzeichnen von Thrombozytenaggregation mit mindestens einer Messküvette (1), mit einer Einrichtung (10) zum Bewegen der zu untersuchenden Flüssigkeit (14) und mit einer Mess- und Auswertungselektronik (8, 9) mit Kontaktelementen, wobei die Messküvette (1) zwei nebeneinander angeordnete, oben offene Teilräume (2, 3) enthält, die nur im unteren Bereich (5) miteinander verbunden sind, und die Einrichtung (10) zum Bewegen der Flüssigkeit eine im Wesentlichen dichtend auf einen der Teilräume (2) aufsetzbare periodisch und abwechselnd einen Unterdruck/Überdruck erzeugende Pumpeinrichtung (11 - 13) ist, **dadurch gekennzeichnet, dass** die Messküvette zwei Elektroden (6) aufweist, die als in den Boden der Messküvette (1) eingesetzte Metallkugeln (6) ausgebildet sind und dass die Mess- und Auswertungselektronik (8, 9) über die Kontaktelemente (7) mit den Elektroden (6) verbunden ist.

2. Aggregometer nach Anspruch 1, **dadurch gekennzeichnet, dass** die Metallkugeln (6) in den Boden der Küvette (1) eingegossen oder eingepresst sind.

3. Aggregometer nach Anspruch 1 oder 2, **dadurch gekennzeichnet, dass** die Metallkugeln (6) aus Edelstahl sind.

4. Aggregometer nach einem der Ansprüche 1 bis 3, **dadurch gekennzeichnet, dass** die Metallkugeln (6) beschichtet sind.

5. Aggregometer nach Anspruch 4, **dadurch gekennzeichnet, dass** die Metallkugeln (6) mit Silber, einem nichtrostenden Material, einem Edelmetall und/oder einem Reagenz beschichtet sind.

6. Aggregometer nach einem der Ansprüche 1 bis 5, **dadurch gekennzeichnet, dass** die Pumpgeschwindigkeit veränderbar ist.

7. Aggregometer nach einem der Ansprüche 1 bis 6, **dadurch gekennzeichnet, dass** die Pumpeinrichtung (10) einen Zylinder (11) und einen Kolben (12) aufweist, der durch einen Exzenterantrieb (13) hin- und her bewegbar ist.

8. Aggregometer nach einem der Ansprüche 1 bis 6, **dadurch gekennzeichnet, dass** die Pumpeinrichtung (10) eine Membranpumpe ist.

9. Aggregometer nach einem der Ansprüche 1 bis 8, **dadurch gekennzeichnet, dass** der Teilraum (2), auf den die Pumpeinrichtung (10) aufsetzbar ist, im Wesentlichen parallele Wände aufweist, während sich der andere Teilraum (3) nach oben hin trichterförmig erweitert.

10. Aggregometer nach einem der Ansprüche 1 bis 9, **dadurch gekennzeichnet, dass** die Kontaktelemente (7) federnd ausgebildet sind.

11. Aggregometer nach einem der Ansprüche 1 bis 10, **dadurch gekennzeichnet, das** mehrere Messküvetten (11) nebeneinander angeordnet sind.

12. Aggregometer nach Anspruch 11, **dadurch gekennzeichnet, dass** die einzelnen Küvetten (1) gleichzeitig mit der Mess- und Auswerteelektronik (8, 9) verbindbar sind, indem die Metallkugeln (6) mithilfe ihrer Kontaktelemente (7) mit der Elektronik (8, 9) verbunden werden, wobei eine oder mehrere Pumpeinrichtungen (10) auf die Messküvetten (1) aufgesetzt und betrieben werden.

13. Aggregometer nach einem der Ansprüche 1 bis 12, **dadurch gekennzeichnet, dass** die Küvetten (1) Spritzgussteile aus blutverträglichen Kunststoffmaterialien sind, die aus Polystyrol, Polymethylmethacrylat und Polyethylen ausgewählt sind.

## Claims

1. Aggregometer for measuring and recording thrombocyte aggregation, having at least one sample cell (1), having a device (10) for moving the liquid (14) to be tested, and having an electronic measuring and evaluation unit (8, 9) with contact elements, wherein the sample cell (1) comprises two adjacent open-top sub-chambers (2, 3) which are connected to one another only in the lower region (5), and the device (10) for moving the liquid is a pump device (11 - 13) which can be seated substantially tightly on one of the sub-chambers (2) and periodically and alternately generates a low pressure/overpressure, **characterised in that** the sample cell has two electrodes (6) which are in the form of metal spheres (6) set into the bottom of the sample cell (1), and **in that** the electronic measuring and evaluation unit (8, 9) is connected to the electrodes (6) by way of the contact elements (7).

2. Aggregometer according to claim 1, **characterised in that** the metal spheres (6) are moulded or pressed into the bottom of the cell (1).

3. Aggregometer according to claim 1 or 2, **characterised in that** the metal spheres (6) are made of stainless steel.

4. Aggregometer according to any one of claims 1 to 3, **characterised in that** the metal spheres (6) are coated.

5. Aggregometer according to claim 4, **characterised in that** the metal spheres (6) are coated with silver, with a non-rusting material, with a noble metal and/or with a reagent.

6. Aggregometer according to any one of claims 1 to 5, **characterised in that** the pump speed is changeable.

7. Aggregometer according to any one of claims 1 to 6, **characterised in that** the pump device (10) has a cylinder (11) and a piston (12) which is movable to and fro by an eccentric drive (13).

8. Aggregometer according to any one of claims 1 to 6, **characterised in that** the pump device (10) is a membrane pump.

9. Aggregometer according to any one of claims 1 to 8, **characterised in that** the sub-chamber (2) on which the pump device (10) can be seated has substantially parallel walls, while the other sub-chamber (3) widens towards the top in the manner of a funnel.

10. Aggregometer according to any one of claims 1 to 9, **characterised in that** the contact elements (7) are resilient.

11. Aggregometer according to any one of claims 1 to 10, **characterised in that** a plurality of sample cells (11) are arranged side by side.

12. Aggregometer according to claim 11, **characterised in that** the individual cells (1) can simultaneously be connected to the electronic measuring and evaluation unit (8, 9) by connecting the metal spheres (6) to the electronic unit (8, 9) with the aid of their contact elements (7), one or more pump devices (10) being seated on the sample cells (1) and being operated.

13. Aggregometer according to any one of claims 1 to 12, **characterised in that** the cells (1) are injection-moulded parts made of blood-compatible plastics materials selected from polystyrene, polymethyl methacrylate and polyethylene.

## Revendications

1. Agrégomètre pour la mesure et l'enregistrement de l'agrégation plaquettaire comportant au moins une cuvette de mesure (1), dotée d'un dispositif (10) pour déplacer le liquide à analyser (14) et un système électronique de mesure et d'évaluation (8, 9) avec éléments de contact, la cuvette de mesure (1) contenant deux chambres partielles (2, 3) ouvertes sur le dessus, disposées côte à côte, qui sont reliées uniquement dans la zone inférieure (5), et le dispositif (10) de déplacement du liquide étant un dispositif de pompage (11 - 13) pouvant essentiellement être placé de façon étanche sur l'une des chambres partielle (2), produisant périodiquement et alternativement, une sous-pression/surpression, **caractérisé en ce que** la cuvette de mesure présente deux électrodes (6) qui sont constituées comme des billes métalliques (6) insérées dans le fond de la cuvette de mesure (1) et **en ce que** le dispositif électronique de mesure et d'évaluation (8, 9) est relié par le biais des éléments de contact (7) aux électrodes (6).

2. Agrégomètre selon la revendication 1, **caractérisé en ce que** les billes métalliques (6) dans la base de la cuvette (1) sont coulées ou comprimés.

3. Agrégomètre selon la revendication 1 ou 2, **caractérisé en ce que** les billes métalliques (6) sont en acier inoxydable.

4. Agrégomètre selon l'une des revendications 1 à 3, **caractérisé en ce que** les billes métalliques (6) sont enrobées.

5. Agrégomètre selon la revendication 4, **caractérisé en ce que** les billes métalliques (6) sont enrobées d'argent, de matériau anti-rouille, d'un métal noble et/ou d'un réactif.

6. Agrégomètre selon l'une des revendications 1 à 5, **caractérisé en ce que** la vitesse de pompage est réglable.

7. Agrégomètre selon l'une des revendications 1 à 6, **caractérisé en ce que** le dispositif de pompage (10) présente un cylindre (11) et un piston (12) qui peut exercer un mouvement de va-et-vient par un système à excentrique (13).

8. Agrégomètre selon l'une des revendications 1 à 6, **caractérisé en ce que** le dispositif de pompage (10) est une pompe membranaire.

9. Agrégomètre selon l'une des revendications 1 à 8, **caractérisé en ce que** la chambre partielle (2) sur laquelle le dispositif de pompage (10) peut être placé présente essentiellement des parois parallèles alors que l'autre chambre partielle (3) s'élargit vers le haut en forme d'entonnoir.

10. Agrégomètre selon l'une des revendications 1 à 9, **caractérisé en ce que** les éléments de contact (7) sont élastiques.

11. Agrégomètre selon l'une des revendications 1 à 10, **caractérisé en ce que** plusieurs cuvettes de mesure (11) sont placées côte à côte.

12. Agrégomètre selon la revendication 11, **caractérisé en ce que** les cuvettes de mesure individuelles (1) peuvent être reliées en même temps au système électronique de mesure et d'évaluation (8, 9), les billes métalliques (6) étant reliées à l'aide de leurs éléments de contact (7) au système électronique (8, 9), un ou plusieurs dispositifs de pompage (10) étant placés et activés sur les cuvettes de mesure (1).

13. Agrégomètre selon l'une des revendications 1 à 12, **caractérisé en ce que** les cuvettes (1) sont des éléments moulés par injection en matières plastiques compatibles avec le sang, qui sont choisies parmi le polystyrène, le polyméthylméthacrylate et le polyéthylène.
